# EUROPÄISCHE PATENTSCHRIFT

(11) **EP 2 033 606 B1**
(45) Veröffentlichungstag und Bekanntmachung des Hinweises auf die Patenterteilung: **10.03.2010**
(21) Anmeldenummer: 07017422.2
(22) Anmeldetag: 05.09.2007
(51) Int. Cl.: A61F 11/12

(54) **Gehörschutzvorrichtung**
Hearing protection device
Dispositif de protection acoustique

(43) Veröffentlichungstag der Anmeldung: 11.03.2009
(73) Patentinhaber: Moldex-Metric AG & Co. KG, 72141 Walddorfhäslach (DE)
(72) Erfinder: Skov, Roman, 70197 Stuttgart (DE)
(74) Vertreter: Grünecker, Kinkeldey, Stockmair & Schwanhäusser Anwaltssozietät

(56) Entgegenhaltungen:
- EP-A- 0 587 925
- EP-A- 0 911 004
- WO-A-98/06363

## Beschreibung

Die Erfindung bezieht sich auf eine Gehörschutzeinrichtung der im Oberbegriff von Anspruch 1 erläuterten Art.

Derartige Gehörschutzeinrichtungen sind in großer Zahl bekannt, beispielsweise aus der im Oberbegriff berücksichtigten EP 911 004 oder der EP 836 841 oder der EP 870 487. Die bekannten Gehörschutzeinrichtungen sind im Wesentlichen nach dem gleichen Prinzip aufgebaut. Sie enthalten einen federnd biegbaren Bügel mit zwei freien Enden, an denen jeweils ein Ohrstöpsel befestigt ist. Der Bügel wird beim Aufsetzen der Gehörschutzeinrichtung etwas auseinander gezogen, so dass die Ohrstöpsel bei der nachfolgenden Entspannung des Bügels fest in den äußeren Gehörgang des Benutzers eingedrückt werden. Die Bügel sind so ausgebildet, dass sie im Gebrauch unterhalb des Kinns, d. h. zwischen Kinn und Hals, ihren Platz finden. Die Bügel enthalten einen vorderen Bereich, der sich bei allen Gehörschutzeinrichtungen der oben beschriebenen Druckschriften über das Kinn und die Wangen bis in die Nähe des Ohrs des Trägers erstreckt und dort unter einem Winkel in einen weiteren Bereich übergeht, der die Verbindung zum Ohr bildet. Der weitere Bereich ist so abgewinkelt, dass er einen Freiraum für das Ohrläppchen des Benutzers schafft, so dass dieses nicht am Bügel reibt, wenn sich der Kopf relativ zum Bügel bewegt. Der Übergang zwischen den Bereichen liegt bei allen Gehörschutzeinrichtungen der oben genannten Druckschriften relativ weit in Richtung auf das Ohr, da nur dort der Freiraum benötigt wird. Zur Umgehung des Ohrläppchens sind in allen oben beschriebenen Druckschriften Winkel zwischen 20 und 30 Grad dargestellt, wobei die EP 911 004 einen Winkel von 30 Grad erwähnt. Dieser relativ große Winkel dient jedoch weniger der Schaffung eines Freiraums für das Ohrläppchen, sondern dazu, die Ohrstöpsel gegen Verschmutzung zu sichern, wenn dieser Gehörschutz auf einer ebenen Unterlage abgelegt wird. Durch diesen Winkel von 30 Grad werden die Ohrstöpsel sicher von der Unterlage abgehoben. Dies funktioniert jedoch nur, wenn der Übergang zwischen dem ersten und dem weiteren Bereich relativ nahe am Ohrstöpsel liegt, da ansonsten das Gewicht der Ohrstöpsel den Bügel kippen würde und der angestrebte Zweck, die Ohrstöpsel von einer Unterlage fern zu halten, nicht erreicht würde.

Bei allen bekannten Gehörschutzeinrichtungen besteht das Problem, dass die Bügel in gewissem Umfange schallleitend sind, was sich unangenehm bemerkbar macht, wenn der Bügel an der Kleidung, beispielsweise am Kragen, reibt. Um dieses Problem zu lösen wurde bereits in der EP 836 841 vorschlagen, den Bügel aus zwei verschieden harten Materialien herzustellen, um eine Unterbrechung der Schallfortleitung zu erreichen. Diese Ausgestaltung ist jedoch relativ kompliziert und somit teuer in der Herstellung, was sich bei Massenprodukten mit begrenzter Lebensdauer doch nachteilig auswirkt.

Der Erfindung liegt somit die Aufgabe zugrunde, eine Gehörschutzeinrichtung zu schaffen, die auf konstruktiv einfache Weise eine Geräuschbelästigung durch ein Anstoßen des Bügels insbesondere an den Kragen und an den Kleidungsstücken verringert.

Die Aufgabe wird durch die im Anspruch 1 angegebenen Merkmale gelöst.

Durch die erfindungsgemäße Ausgestaltung wird die Geräuschbelästigung durch eine vergrößerte Halsfreiheit, d. h. einen vergrößerten Abstand zwischen Bügel und Hals im Gebrauch, verringert, wobei der Bügel in zwei deutlich unterscheidbare Bereiche eingeteilt wird; der Wangenbereich sorgt für den notwendigen vertikalen Abstand zwischen dem Ohr und dem Kinnbereich, damit der Kinnbereich derart abgewinkelt werden kann, dass er sich eng unter das Kinn legt und somit die Gefahr eines Kontakts mit dem Kragen eines Kleidungsstückes wesentlich vermindert wird. Diese Ausgestaltung führt dazu, dass der Verlauf des Bügels im Kinn- und Wangenbereich der Tendenz nach an den Verlauf eines Unterkieferknochens eines erwachsenen Menschen angepasst ist, wobei der Kinnbereich dem Unterkieferkörper und der Wangenbereich dem Unterkieferast folgt. Durch diese Ausgestaltung wird weiterhin der Schwerpunkt der gesamten Gehörschutzeinrichtung im Gebrauch etwas in Richtung Ohr bzw. Hals verlagert, so dass der im Wesentlich horizontal vorstehende Kinnbereich besser durch die Reibung der Ohrstöpsel im Ohr in seiner horizontalen Lage gehalten werden kann und sich auch bei Erschütterungen nicht selbsttätig nach unten in Richtung auf den Kragen des Benutzers verschwenkt.

Vorteilhafte Weiterbildungen der Erfindung sind den Unteransprüchen zu entnehmen.

Ein Ausführungsbeispiel der Erfindung wird nachfolgend anhand der Zeichnungen näher erläutert. Es zeigen:
- Fig. 1: eine erfindungsgemäße Gehörschutzeinrichtung in perspektivischer Darstel- lung,
- Fig. 1A, 1B, 1C: Schnittansichten I-I, II-II und III-III aus Fig.1,
- Fig. 2: die Gehörschutzeinrichtung nach Fig. 1 in Seitenansicht,
- Fig. 3: die Gehörschutzeinrichtung gemäß Fig. 1 in Draufsicht,
- Fig. 4: die Gehörschutzeinrichtung gemäß Fig. 1 in Vorderansicht, und
- Fig. 5: die Gehörschutzeinrichtung gemäß Fig. 1 in Gebrauch.

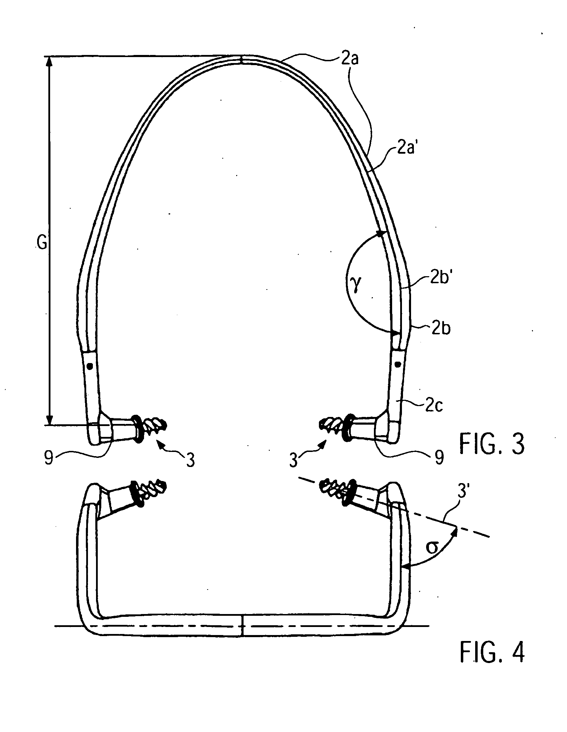

Aus den Figuren ist in schematischer Darstellung eine erfindungsgemäße Gehörschutzeinrichtung 1 ersichtlich, die einen Bügel 2 aufweist, der aus einem der üblichen Materialien, insbesondere Kunststoff, gefertigt ist, die auch bisher bereits für die Bügel verwendet wurden. Der Bügel 2 ist als langgestrecktes, in Draufsicht im Wesentlichen hufeisenförmiges (Fig. 3) Element ausgebildet und enthält an seinen beiden freien Enden jeweils einen Träger 3, auf dem ein Ohrstöpsel 4, bevorzugt aus geräuschhemmenden Materialien, insbesondere einem Kunststoffschaum, befestigt ist. Die Befestigung der Ohrstöpsel 4 ist bevorzugt lösbar, damit diese bei Verschleiß oder Verschmutzung ausgetauscht werden können. Die Ohrstöpsel 4 sind an die anatomischen Gegebenheiten im äußeren Gehörgang von erwachsenen Benutzern angepasst.

Im dargestellten Ausführungsbeispiel enthält der Träger 3 ein Gewindeteil, bevorzugt mit großer Steigung und spitzwinkligen, relativ scharfkantigen Gängen, der in eine im Ohrstöpsel 4 vorgesehene Öffnung schraubend eingebracht wird. Dabei muss die Öffnung des Ohrstöpsels nicht notwendiger Weise ein Gegengewinde tragen, vielmehr drückt sich der Gewindegang des Trägers in das Material des Ohrstöpsels 4 hinein, so fern der Durchmesserunterschied zwischen der Öffnung im Ohrstöpsel 4 und dem Gewinde des Trägers ausreichend groß ist. Weiterhin verläuft das Gewinde des Trägers 3 bevorzugt konisch oder enthält eine Einstichspitze in der Art von Holzschrauben. Die Befestigung der Ohrstöpsel 4 mittels Gewinde ist jedoch auch bei herkömmlich geformten Bügeln einsetzbar.

Der Träger 3 ist unter einem anatomisch bedingten, von 90° abweichenden, räumlichen Winkel nach oben und leicht nach vom (Richtung im Gebrauch) am Bügel 2 befestigt und definiert eine Drehachse 3', unter der sich der Ohrstöpsel 4 in Ohr des Benutzers drehen kann, wenn der Bügel 2 im Gebrauch verschwenkt wird.

Im unbelasteten Zustand ist der Bügel 1 so geformt, dass sich die Ohrstöpsel 4 in einem Abstand gegenüber liegen, der geringer ist als die Kopfbreite eines Benutzers im Gebrauch. Zum Gebrauch der Gehörschutzeinrichtung 1 muss der Bügel somit auseinander gezogen werden, bis die Ohrstöpsel 4 in die Ohren des Benutzers eingesetzt werden können. Nach dem Loslassen des Bügels federt dieser in einer Stellung zurück, in der die Ohrstöpsel 4 in die Ohren des Benutzers eingedrückt werden.

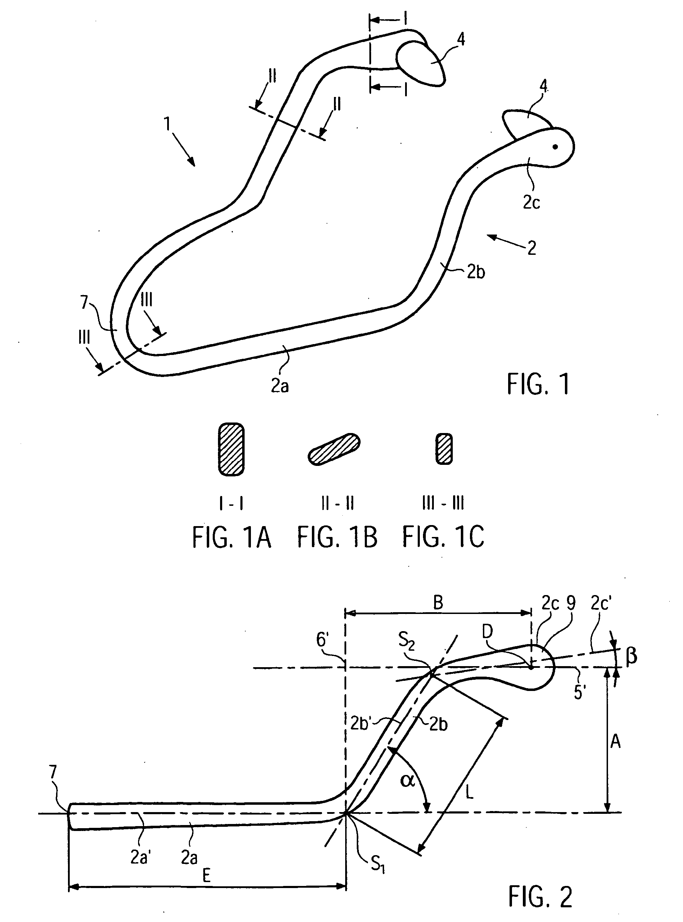

In den Figuren 1 bis 4 ist der Bügel 2 im entspannten, unbelasteten Zustand gezeigt, wobei die nachfolgend beschriebenen Maß- und Winkelverhältnisse sich ebenfalls auf den unbelasteten, d. h. unverformten Zustand beziehen.

Der Bügel 2 der erfindungsgemäßen Gehörschutzeinrichtung 1 enthält wenigstens einen Kinnbereich 2a und einen Wangenbereich 2b sowie bevorzugt zusätzlich einen ohrnahen Verbindungsbereich 2c. Diese drei Bereiche 2a bis 2c sind deutlich voneinander unterscheidbar durch ihre relativ Lage zueinander.

Der Kinnbereich 2a weist eine Mittellinie 2a' auf, und erstreckt sich in einer Ebene. In Draufsicht ist der Kinnbereich 2a im Wesentlich parabelförmig bzw. spitzzulaufend gekrümmt, angepasst an die Krümmungslinie eines menschlichen Unterkiefers. Beidseitig an den Kinnbereich 2a schließt sich jeweils ein Wangenbereich 2b an. Der Wangenbereich 2b erstreckt sich vom Kinnbereich 2a nach schräg oben, wobei die Mittellinien 2b' des Wangenbereichs 2b zur Mittellinie 2a' des jeweils anliegenden Teils des Kinnbereiches 2a einen Winkel α aufweist. Der Winkel α liegt zwischen 35° und 90°, bevorzugt 45° bis 70° und insbesondere bevorzugt zwischen 50° und 60°. In einem besonders bevorzugten Ausführungsbeispiel beträgt der Winkel α zwischen 53 und 58°.

Der Wangenbereich 2b ist bevorzugt gerade und so angeordnet und bemessen, dass eine Verlängerung einer Mittellinie 2a' des Kinnbereichs 2a und eine dazu parallele Linie 5', die durch einen Durchdringungspunkt D der Drehachse 3' durch den Bügel 2 führt, einen Abstand A zueinander aufweisen, der zwischen 30 bis 80 mm, bevorzugt zwischen 40 bis 60 mm und insbesondere bevorzugt zwischen 45 bis 55 mm beträgt. In einem bevorzugten Ausführungsbeispiel beträgt dieser Abstand 45 mm.

Weiterhin weist der Durchdringungspunkt D der Drehachse 3' durch den Bügel 2 von einer senkrecht zur Mittellinie 2a' des Kinnbereichs 2a durch ihren Schnittpunkt S1 mit der Mittellinie 2b' des Wangenbereichs 2b verlaufenden Linie 6' einen Abstand B von 55 bis 90 mm, bevorzugt 60 bis 80 mm und insbesondere bevorzugt von 65 bis 75 mm auf.

Der Wangenbereich 2b weist entlang seiner Mittellinie 2b' zwischen dem Schnittpunkt S1der Mittellinie 2b' mit der Mittellinie 2a' des Kinnbereichs 2a und einem Schnittpunkt S2 mit der Mittellinie 2c' des Verbindungsbereichs 2c eine Länge L auf, die zwischen 40 bis 65 mm, bevorzugt zwischen 45 mm bis 55 mm und insbesondere bevorzugt zwischen 47 mm bis 53 mm beträgt. In einem bevorzugten Ausführungsbeispiel beträgt die Länge L 50 mm.

Der Verbindungsbereich 2c erstreckt sich vom Wangenbereich 2b im Wesentlichen parallel zum Kinnbereich 2a, wobei die Abweichungen von der Parallelität weniger als 5 Grad betragen. Dies bedeutet, dass der Winkel β zwischen der Mittellinie 2c' des Verbindungsbereiches 2c und der Mittellinie 2a' des Kinnbereichs 2a bzw. der dazu parallelen Linie 5' zwischen 0 und 5 Grad beträgt.

Die einzelnen Bereiche 2a, 2b, 2c gehen über jeweils eine Rundung ineinander über. Die Erstreckung E des Kinnbereichs 2a zwischen einer Verbindungslinie zwischen den Schnittpunkten S1 und der am weitesten von den Ohrstöpseln 5 entfernten Mitte 7 des Kinnbereichs 2a beträgt zwischen 30 mm und 100 mm, bevorzugt 60±30 mm. Die Gesamtlänge G der Gehörschutzeinrichtung 1 zwischen den Ohrstöpseln 4 (am Durchdringungspunkt D) und der Mitte 7 in Draufsicht beträgt bevorzugt etwa 135 bis 165 mm, und insbesondere bevorzugt 135 bis 155 mm, und besonders bevorzugt 145 mm.

Der Bügel 2 weist im allgemeinen einen langgestreckt-rechteckigen Materialquerschnitt auf, wobei jedoch die Orientierung der längeren Seite des Querschnitts, sowie ggf. die Länge und Breite, im Verlauf des Bügels durch die unterschiedlichen Bereiche wechselt. So ist im Verbindungsbereich 2c der Querschnitt mit seiner langen Seite vertikal angeordnet, wie dies in Fig. 1A gezeigt ist. Im Wangenbereich 2b ist der Querschnitt im Wesentlichen horizontal bis schräg, d.h. leicht nach innen ansteigend, wie dies in Fig. 1C gezeigt ist. Der Kinnbereich 2a zeigt wiederum den im Wesentlichen vertikal verlaufenden Querschnitt (Fig. 1C). Der Querschnitt im Kinnbereich 2a, insbesondere im Bereich der Mitte 7, ist mit verringerter Breite (zwischen den längeren Seiten) ausgebildet, wobei sich die Verformung beim Auseinanderziehen zum Anlegen des Gehörschutzes 1 im Kinnbereich konzentriert. Die Übergänge zwischen den verschiedenen Anordnungen gemäß den Figuren 1A bis 1C verlaufen fließend und ohne scharfe Kanten, die potentielle Bruchstellen bieten könnten.

Die Hufeisenform des Bügels 2 in Draufsicht (Fig. 3) wird weiterhin durch einen horizontalen Winkel χ (Draufsicht in Fig. 3) unterstützt, unter dem die Mittellinien 2a' und 2b' von Kinn-und Wangenbereich 2a, 2b aufeinander treffen. Dieser Winkel χ beträgt zwischen 135 bis 170 und bevorzugt zwischen 140 und 170 Grad und insbesondere bevorzugt zwischen 150 bis 170 Grad, und bevorzugt 165 ±1 bis 2 Grad.

Die Drehachse 3' durch den Träger 3 verläuft unter einem in Vorderansicht schräg aufwärts gerichteten Winkel δ, von etwa 70 Grad. An der Befestigung des Trägers 3 am Verbindungsbereich 2c ist der Verbindungsbereich 2c in vertikaler Richtung zu einer Ansatzstelle 9 verbreitert, so dass sich die Drehachse 3' durch den verbreiterten Bereich hindurch erstreckt.

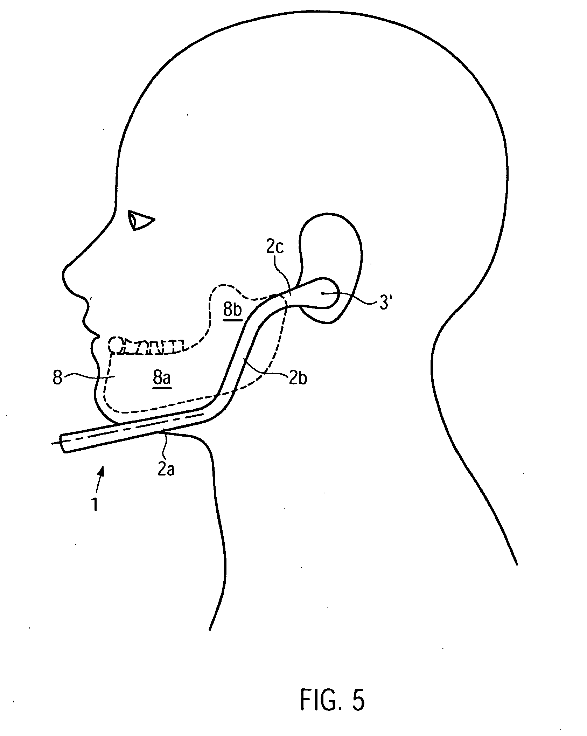

Fig. 5 zeigt die Position der erfindungsgemäßen Gehörschutzeinrichtung am Benutzer. Gestrichelt eingezeichnet ist die Lage und ungefähre äußere Form eines Unterkiefers 8 mit Unterkieferkörper 8a und Unterkieferast 8b. Es ist deutlich zu sehen, dass sich die erfindungsgemäße Gehörschutzeinrichtung 1 an den Verlauf bzw. die äußere Form des Unterkiefers 8 anpasst, wobei der Kinnbereich 2a im Wesentlichen parallel zur unteren Begrenzung des Unterkieferkörpers 6a ausgerichtet werden kann, wobei in dieser Stellung der Wangenbereich 2b dem Anstieg des Unterkieferastes 8b folgt.

Der Verbindungsbereich 2c überbrückt dann den Abstand zwischen der ungefähren Lage des Unterkiefergelenks und dem äußeren Ohrkanal des Benutzers. Wie Fig. 5 deutlich zeigt, bietet die erfindungsgemäße Gehörschutzeinrichtung 1 eine große Halsfreiheit, d. h. der Kinnbereich 2a erstreckt sich in der bevorzugten, mit durchgezogenen Linien gezeigten Position sehr weit oberhalb eines eventuell vorhandenen Kragens, so dass ein geräuschübertragendes Reiben an der Kleidung auch bei einer starken Neigung des Kopfes weniger wahrscheinlich ist. Durch die erfindungsgemäße Ausgestaltung wird weiterhin der Schwerpunkt näher in Richtung auf die Drehachse 3 verlagert, so dass auch bei den üblichen Erschütterungen weniger die Gefahr besteht, dass die Gehörschutzeinrichtung sich selbsttätig nach unten verschwenkt.

In Abwandlung des beschriebenen und gezeichneten Ausführungsbeispiels kann die Befestigung der Ohrstöpsel mit Hilfe des Gewindes am Träger auch bei Gehörschutzeinrichtungen eingesetzt werden, die einen konventionellen Bügel aufweisen. Weiterhin kann der Verbindungsbereich ggf. entfallen und der Wangenbereich vom Kinnbereich direkt zum Träger führen. Auch die speziell angegebenen, bevorzugten Abmessungen können innerhalb des Bereichs für verschiedene Kopfgrößen variiert werden. Die Querschnittsformen können ebenfalls angepasst werden.

## Patentansprüche

1. Gehörschutzeinrichtung (1) mit einem federnd biegbaren Bügel (2), an dessen freien Enden jeweils ein Ohrstöpsel (4) angeordnet ist, wobei der Bügel (2) einen ersten Bereich (2a) aufweist, dessen Mittellinie (2a') sich unter einem Winkel (α) an einen weiteren Bereich (2b) anschließt, und der Bügel (2) im Gebrauch um eine sich durch die Ohrstöpsel (4) erstreckende Drehachse (3') im Ohr verschwenkbar ist, **dadurch gekennzeichnet, dass** der erste Bereich als Kinnbereich (2a) und der weitere Bereich als Wangenbereich (2b) ausgebildet ist, der Winkel (α) zwischen 35° und 90°, bevorzugt 45° bis 70°, und insbesondere bevorzugt zwischen 50° und 60° beträgt und der Wangenbereich (2b) eine derartige Länge (L) aufweist, dass eine Verlängerung der Mittellinie (2a') des Kinnbereichs (2a) von einer dazu parallelen Linie (5'), die durch den Durchdringungspunkt (D) der Drehachse (3') durch den Bügel (2) führt, einen Abstand (A) von 30 bis 80 mm, bevorzugt von 40 bis 60 mm, und insbesondere bevorzugt von 45 bis 55 mm aufweist.

2. Gehörschutzeinrichtung (1) nach Anspruch 1, **dadurch gekennzeichnet, dass** der Bügel (2) einen Verbindungsbereich (2c) zwischen dem Wangenbereich (2b) und den Ohrstöpseln (4) aufweist, dessen Mittellinie (2c') im wesentlichen parallel zur Mittellinie (2a') des Kinnbereichs (2a), mit einer Abweichung von weniger als 5°, verläuft.

3. Gehörschutzeinrichtung (1) nach Anspruch 1 oder 2, **dadurch gekennzeichnet, dass** der Durchdringungspunkt (D) der Drehachse (3') durch den Bügel (2) einen Abstand (B) von 55 bis 90 mm, bevorzugt 60 bis 80 mm und insbesondere bevorzugt von 65 bis 75 mm von einer senkrecht zur Mittellinie (2a') des Kinnbereichs (2a) am Schnittpunkt (S1) mit der Mittellinie (2b') des Wangenbereichs (2b) verlaufenden Linie (6') aufweist.

4. Gehörschutzeinrichtung (1) nach einem der Ansprüche 1 bis 3, **dadurch gekennzeichnet, dass** der Wangenbereich (2b) im wesentlichen geradlinig verläuft.

5. Gehörschutzeinrichtung (1) nach einem der Ansprüche 1 bis 4, **dadurch gekennzeichnet, dass** der Wangenbereich (2b) eine Länge (L) von 40 mm bis 65 mm, bevorzugt 45 mm bis 55 mm und insbesondere bevorzugt 47 mm bis 53 mm aufweist.

6. Gehörschutzeinrichtung (1) nach einem der Ansprüche 1 bis 5, **dadurch gekennzeichnet, dass** der Kinnbereich (2a) im wesentlichen in einer Ebene verläuft.

7. Gehörschutzeinrichtung (1) nach einem der Ansprüche 1 bis 6, **dadurch gekennzeichnet, dass** der Kinnbereich (2a) mit einer Ausrundung in den Wangenbereich (2b) übergeht.

8. Gehörschutzeinrichtung (1) nach einem der Ansprüche 1 bis 7, **dadurch gekennzeichnet, dass** die Länge (E) des Kinnbereich (2a) vom vorderen Ende (7) bis zu einer Verbindungslinie zwischen den Schnittpunkten (S1) der Mittellinien (2a', 2b') des Kinn- und des Wangenbereichs (2a, 2b) zwischen 60 mm und 100 mm beträgt.

9. Gehörschutzeinrichtung (1) nach einem der Ansprüche 1 bis 8, **dadurch gekennzeichnet, dass** der Wangenbereich (2b) mit einer Ausrundung in einen Verbindungsbereich (2c) zu den Ohrstöpseln (4) übergeht.

10. Gehörschutzeinrichtung (1) nach einem der Ansprüche 1 bis 9, **dadurch gekennzeichnet, dass** der Kinnbereich (2a) parabelförmig gekrümmt ist.

11. Gehörschutzeinrichtung (1) nach einem der Ansprüche 1 bis 10, **dadurch gekennzeichnet, dass** sich die Mittellinie (2b') des Wangenbereichs (2b) unter einem horizontalen Winkel (χ) von 130° bis 185° an die Mittellinie (2a') des Kinnbereichs (2a) anschließt.

12. Gehörschutzeinrichtung (1) nach einem der Ansprüche 1 bis 11, **dadurch gekennzeichnet, dass** die Ohrstöpsel (4) lösbar auf einem Träger (3) befestigt sind.

13. Gehörschutzeinrichtung (1) nach einem der Ansprüche 1 bis 12, **dadurch gekennzeichnet, dass** die Ohrstöpsel (4) lösbar auf einen Gewindeträger (3) befestigt sind.

## Claims

1. An ear protector (1) comprising a resilient band (2), the free ends of which each have arranged thereon an earplug (4), said band (2) including a first area (2a) whose centre line (2a') merges under an angle (α) with an additional area (2b), and said band (2) being pivotable, when in use, in the user's ears about an axis of rotation (3') extending through the earplugs (4), **characterized in that** the first area is configured as a chin area (2a) and the additional area as a cheek area (2b), that the angle (α) ranges from 35° to 90°, preferably from 45° to 70° and even more preferably from 50° to 60°, and that the length (L) of the cheek area (2b) is dimensioned such that an extension of the centre line (2a') of the chin area (2a) and a line (5'), which is parallel to said centre line (2a') and which extends through the point of penetration (D) of the axis of rotation (3') through the band (2), are spaced apart at a distance (A) which ranges from 30 to 80 mm, preferably from 40 to 60 mm and even more preferably from 45 to 55 mm.

2. An ear protector (1) according to claim 1, **characterized in that** the band (2) includes a connection area (2c) between the cheek area (2b) and the earplugs (4), the centre line (2c') of said connection area (2c) extending substantially parallel to the centre line (2a') of the chin area (2a) with a deviation of less than 5°.

3. An ear protector (1) according to claim 1 or 2, **characterized in that** the point of penetration (D) of the axis of rotation (3') through the band (2) is spaced apart from a line (6'), which extends at right angles to the centre line (2a') of the chin area (2a) through the point of intersection (S1) of said centre line (2a') with the centre line (2b') of the cheek area (2b), at a distance (B) which ranges from 55 to 90 mm, preferably from 60 to 80 mm and even more preferably from 65 to 75 mm.

4. An ear protector (1) according to one of the claims 1 to 3, **characterized in that** the cheek area (2b) extends substantially straight.

5. An ear protector (1) according to one of the claims 1 to 4, **characterized in that** the cheek area (2b) has a length (L) of 40 mm to 65 mm, preferably 45 mm to 55 mm and even more preferably 47 mm to 53 mm.

6. An ear protector (1) according to one of the claims 1 to 5, **characterized in that** the chin area (2a) extends essentially in one plane.

7. An ear protector (1) according to one of the claims 1 to 6, **characterized in that** the chin area (2a) merges with the cheek area (2b) via a curved portion.

8. An ear protector (1) according to one of the claims 1 to 7, **characterized in that** the length (E) of the chin area (2a) is between 60 mm and 100 mm between the front end (7) and a connection line extending between the points of intersection (S1) of the centre lines (2a', 2b') of the chin and cheek areas (2a, 2b).

9. An ear protector (1) according to one of the claims 1 to 8, **characterized in that** the cheek area (2b) merges via a curved portion with a connection area (2c) to the earplugs (4).

10. An ear protector (1) according to one of the claims 1 to 9, **characterized in that** the chin area (2a) is bent into a parabola shape.

11. An ear protector (1) according to one of the claims 1 to 10, **characterized in that** the centre line (2b') of the cheek area (2b) merges with the centre line (2a') of the chin area (2a) under a horizontal angle (χ) of 130° to 185°.

12. An ear protector (1) according to one of the claims 1 to 11, **characterized in that** the earplugs (4) are releasably fixed to a support (3).

13. An ear protector (1) according to one of the claims 1 to 12, **characterized in that** the earplugs (4) are releasably fixed to a threaded support (3).

## Revendications

1. Dispositif de protection acoustique (1) avec une branche élastiquement flexible (2) aux extrémités libres de laquelle respectivement un obturateur d'oreille (4) est disposé, la branche (2) présentant une première zone (2a), dont la ligne médiane (2a') est en jonction, avec un angle (α), avec une autre zone (2b), et la branche (2) pouvant pivoter dans l'oreille autour d'un axe de rotation (3') s'étendant à travers les obturateurs d'oreille (4) lors de l'utilisation, **caractérisé en ce que** la première zone est réalisée en tant que zone du menton (2a) et l'autre zone en tant que zone des joues (2b), l'angle (α) étant compris entre 35° et 90°, de préférence entre 45° à 70°, et de manière particulièrement préférée entre 50° et 60° et la zone des joues (2b) présentant une longueur (L) faisant qu'un prolongement de la ligne médiane (2a') de la zone du menton (2a) d'une ligne (5') parallèle à celle-ci passant par le point de traversée (D) de l'axe de rotation (3') à travers la branche (2), présente un espacement (A) allant de 30 à 80 mm, de préférence de 40 à 60 mm, et de manière particulièrement préférée de 45 à 55 mm.

2. Dispositif de protection acoustique (1) selon la revendication 1, **caractérisé en ce que** la branche (2) présente une zone de liaison (2c) entre la zone des joues (2b) et les obturateurs d'oreille (4), dont la ligne médiane (2c') s'étend essentiellement parallèlement à la ligne médiane (2a') de la zone du menton (2a), avec une divergence de moins de 5°.

3. Dispositif de protection acoustique (1) selon la revendication 1 ou 2, **caractérisé en ce que** le point de traversée (D) de l'axe de rotation (3') à travers la branche (2) présente un espacement (B) allant de 55 à 90 mm, de préférence de 60 à 80 mm, et de manière particulièrement préférée de 65 à 75 mm, par rapport à une ligne (6') s'étendant perpendiculairement par rapport à la ligne médiane (2a') de la zone du menton (2a) au point d'intersection (S1) avec la ligne médiane (2b') de la zone des joues (2b).

4. Dispositif de protection acoustique (1) selon l'une quelconque des revendications 1 à 3, **caractérisé en ce que** la zone des joues (2b) s'étend essentiellement en ligne droite.

5. Dispositif de protection acoustique (1) selon l'une quelconque des revendications 1 à 4, **caractérisé en ce que** la zone des joues (2b) présente une longueur (L) allant de 40 mm à 65 mm, de préférence de 45 mm à 55 mm, et de manière particulièrement préférée de 47 mm à 53 mm.

6. Dispositif de protection acoustique (1) selon l'une quelconque des revendications 1 à 5, **caractérisé en ce que** la zone des joues (2a) s'étend essentiellement dans un plan.

7. Dispositif de protection acoustique (1) selon l'une quelconque des revendications 1 à 6, **caractérisé en ce que** la zone du menton (2a) se transforme avec un arrondi en zone des joues (2b).

8. Dispositif de protection acoustique (1) selon l'une quelconque des revendications 1 à 7, **caractérisé en ce que** la longueur (E) de la zone du menton (2a), depuis l'extrémité avant (7) jusqu'à une ligne de jonction entre les points d'intersection (S1) des lignes médianes (2a', 2b') de la zone du menton et des joues (2a, 2b), est comprise entre 60 mm et 100 mm.

9. Dispositif de protection acoustique (1) selon l'une quelconque des revendications 1 à 8, **caractérisé en ce que** la zone des joues (2b) se transforme avec un arrondi en une zone de liaison (2c) vers les obturateurs d'oreille (4).

10. Dispositif de protection acoustique (1) selon l'une quelconque des revendications 1 à 9, **caractérisé en ce que** la zone du menton (2a) est courbée en forme de parabole.

11. Dispositif de protection acoustique (1) selon l'une quelconque des revendications 1 à 10, **caractérisé en ce que** la ligne médiane (2b') de la zone du menton (2b) est en jonction, avec un angle horizontal (χ) allant de 130° à 185°, avec la ligne médiane (2a') de la zone du menton (2a).

12. Dispositif de protection acoustique (1) selon l'une quelconque des revendications 1 à 11, **caractérisé en ce que** les obturateurs d'oreille (4) sont fixés de manière amovible sur un support (3).

13. Dispositif de protection acoustique (1) selon l'une quelconque des revendications 1 à 12, **caractérisé en ce que** les obturateurs d'oreille (4) sont fixés de manière amovible sur un support fileté (3).
